# EUROPÄISCHE PATENTSCHRIFT

(11) **EP 0 689 798 B1**
(45) Veröffentlichungstag und Bekanntmachung des Hinweises auf die Patenterteilung: **18.10.2000**
(21) Anmeldenummer: 94810388.2
(22) Anmeldetag: 30.06.1994
(51) Int. Cl.: A61B 17/60, A61B 17/58

(54) **Vorrichtung zum Verbinden von Wirbelknochen**
Device for connecting vertebrae
Dispositif pour connecter des vertèbres

(43) Veröffentlichungstag der Anmeldung: 03.01.1996
(73) Patentinhaber: Sulzer Orthopädie AG, 6340 Baar (CH)
(72) Erfinder: Sebastián Bueno, D. César, Dr., ES-29016 Málaga (ES); Abad Rico, D. José Ignacio, Dr., ES-29005 Málaga (ES); Yurtsever, Mustafa, CH-8280 Kreuzlingen (CH); Baur, Nikolaus, CH-8932 Mettmenstetten (CH)
(74) Vertreter: Sulzer Management AG

(56) Entgegenhaltungen:
- EP-A- 0 330 881
- EP-A- 0 384 001
- EP-A- 0 468 264
- EP-A- 0 469 304
- DE-A- 4 110 002

## Beschreibung

Die Erfindung betrifft eine Vorrichtung zum Verbinden von Wirbelknochen gemäss dem Oberbegriff von Anspruch 1.

Es ist bekannt, bei deformierten oder degenerierten Wirbelsäulen operativ eine korrigierende Vorrichtung mittels Verbindungsmitteln an mindestens zwei Wirbeln einer Wirbelsäule zu befestigen, um dadurch der Wirbelsäule eine erhöhte Stabilität zu verleihen.

Aus der EP 0384001 A1 ist eine korrigierende Vorrichtung bekannt, die einen länglichen, biegbaren Stab mit Verbindungsmittel an mehreren Wirbelknochen einer Wirbelsäule befestigt. Diese Vorrichtung weist ein Verbindungsglied mit einer Öffnung für die Aufnahme eines Teils eines länglichen Stabes auf. Das Verbindungsglied weist eine weiteren Verbindungsbereich auf, um das Verbindungsglied mittels Knochenschrauben an einen Wirbelknochen zu befestigen. In einer Ausführungsform weist das Verbindungsglied eine Schraube auf, mit welcher der längliche Stab im Verbindungsglied fixierbar ist.

Dieses Verbindungsglied weist den Nachteil auf, dass sich eine Relativbewegung zwischen Verbindungsglied und dem biegbaren Stab ergeben kann, wenn grosse Kräfte anliegen.

Eine Vorrichtung gemäss dem Oberbegriff des Anspruchs 1 ist z.B. aüs der DE-C1-41 10 002 bekannt. Dort wird eine Dreipunktlagerung des Stabes erreicht. Der Stab liegt auf zwei Kanten auf, die dritte Lagerung erfolgt durch eine Klemmschraube.

Der vorliegenden Erfindung liegt die Aufgabe zugrunde, eine Vorrichtung nach dem Oberbegriff des Anspruchs 1 derart weiterzubilden, dass eine verbesserte Verbindung erzielt wird.

Diese Aufgabe wird erfindungsgemäss gelöst durch den Gegenstand mit den in Anspruch 1 definierten Merkmalen. Weitere vorteilhafte Weiterbildungen der Erfindung ergeben sich aus den Unteransprüchen.

Die Vorteile der Erfindung sind darin zu sehen, dass das Verbindungsglied eine Durchbrechung zur Aufnahme eines länglichen Stabes mit kreisförmigen Querschnitt aufweist, deren Innenfläche derart ausgestaltet ist, dass Teilbereiche der Innenfläche mit dem Stab eine Wirkverbindung eingehen.

Die Durchbrechung weist dazu eine innere Begrenzungsfläche auf, die aus mehreren gekrümmten Teilflächen mit mindestens drei unterschiedlichen Krümmungsradien besteht. Diese Krümmungsradien sind je nach Teilfläche grosser oder kleiner als der Krümmungsradius des Stabes, sodass sich zwischen der Begreuzungsfläche der Durchbrechung und dem Stab eine Dreipunktlagerung ergibt, wobei der Stab an zwei Auflagebereichen an der inneren Begrenzungsfläche anliegt, und der dritte Aüflagebereich durch ein auf den Stab wirkendes Verbindungsmittel, wie eine Schraube, erzeugt wird.

Ein weiterer Vorteil einer Ausführungsform der Erfindung ist darin zu sehen, dass im Verbindungsglied, entlang der Ausnehmung, mehr als eine Schraube zur Befestigung des Stabes angeordnet sein können, was dem Stab einen besonders festen Halt verleiht.

Die Figuren zeigen verschiedene Ausführungsbeispiele der Erfindung. Es zeigen:
- Fig. 1a-1c: eine perspektivische Ansicht eines Verbindungsgliedes;
- Fig. 1d: eine Detailansicht der Befestigungsmittel eines Verbindungsgliedes;
- Fig. 2a: eine Seitenansicht eines Verbindungsgliedes;
- Fig. 2c: eine Darstellung des Verlauf der inneren Begrenzungsfläche;
- Fig. 2d: einen im Verbindungsglied eingespannten Stab;
- Fig. 3: einen Querschnitt durch einen Wirbelknochen;
- Fig. 4: einen Längsschnitt durch ein Verbindungsglied gemäss Fig. 1c.

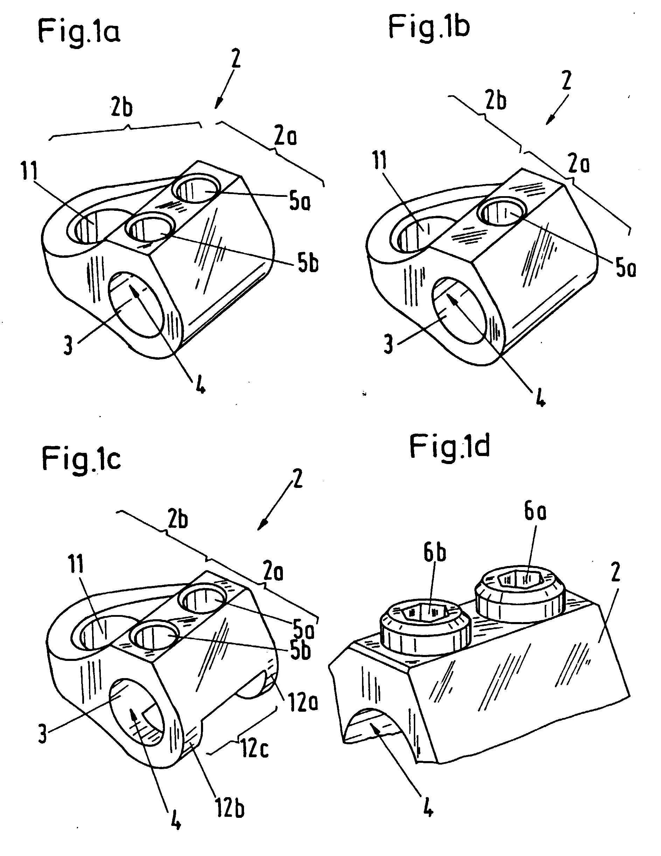

Fig. 1a zeigt ein Verbindungsglied 2, das aus einem Aufnahmeteil 2a sowie einem Verbindungsteil 2b besteht. Das Aufnahmeteil 2a weist eine durchgehende Durchbrechung 3 auf, in die ein Stab 1 einführbar ist, welcher mittels in die Ausnehmung 5a, 5b einführbarer Befestigungsmittel 6a, 6b mit dem Aufnahmeteil 2a verbindbar ist. Die Durchbrechung 3 weist eine innere Begrenzungsfläche 4 auf. Das Verbindungsteil 2b weist eine Durchbrechung 11 auf, durch welche eine Knochenschraube 8 einführbar ist, um das Verbindungsteil 2b an einem Wirbelknochen 7 einer Wirbelsäule zu verankern.

In Fig. 1b ist ein weiteres Verbindungsglied 2 dargestellt, das ähnlich wie in Fig. 1a dargestellt ausgestaltet ist, jedoch nur eine Ausnehmung 5a zur Aufnahme eines Befestigungsmittels 6a aufweist.

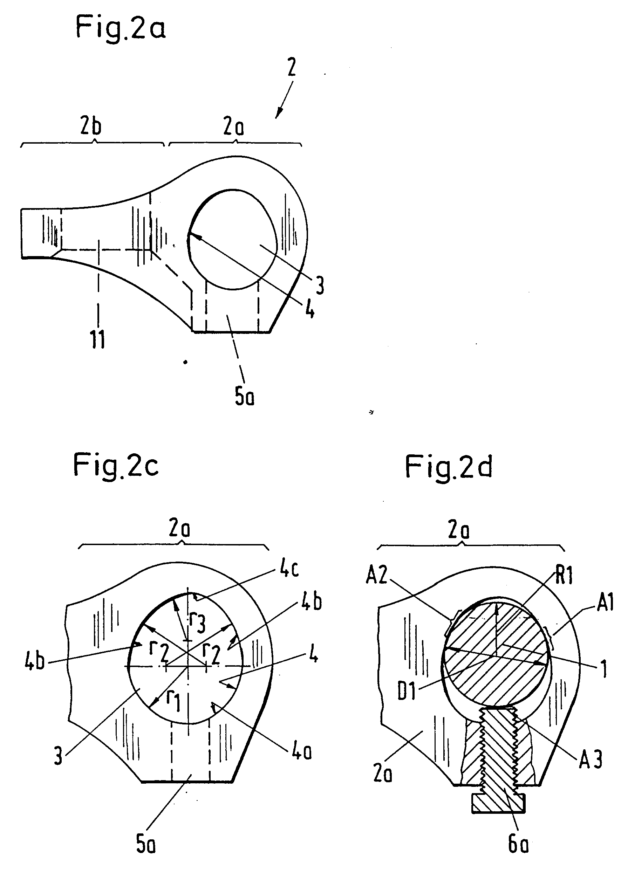

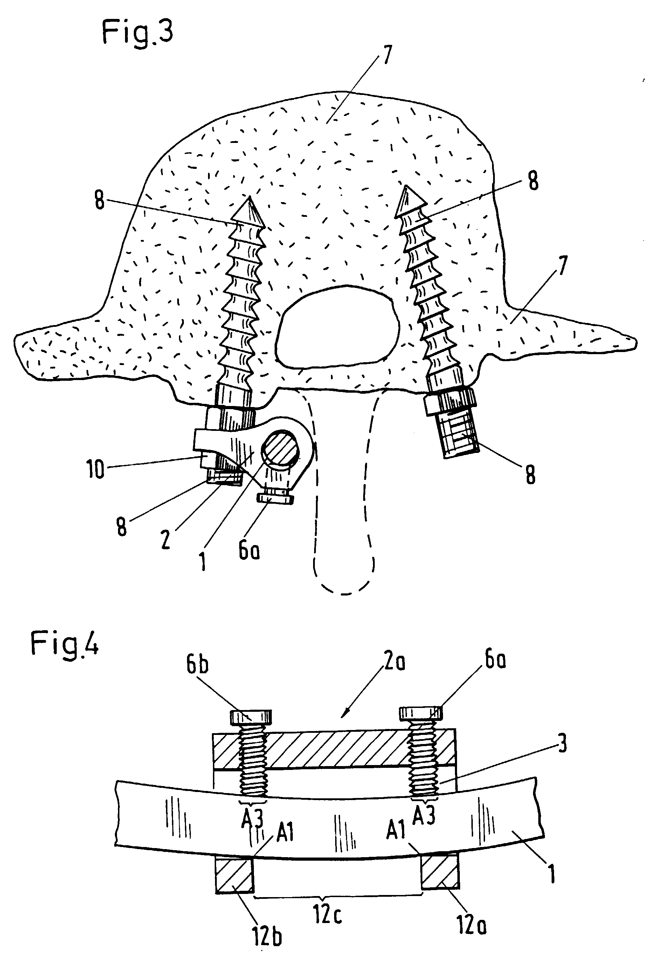

Das Verbindungsglied 2 gemäss Fig. 1c weist im Vergleich zu Fig. 1a eine weitere Durchbrechung 12c der inneren Begrenzungsfläche 4 auf, die gegenüber den Öffnungen der Ausnehmungen 5a, 5b liegt, und in Längsrichtung der Durchbrechung 3 verläuft. Dabei werden zwei Auflageteile 12a, 12b gebildet. Fig. 4 zeigt einen Längsschnitt durch das Aufnahmeteil 2a, woraus die Ausdehnung der Durchbrechung 12c ersichtlich ist, die beidseitig durch die Auflageteile 12a, 12b begrenzt wird. Ein Vorteil der dargestellten Ausführungsform ist darin zu sehen, dass, insbesondere bei einem gekrümmten Stab 1, sich zwei Auflagebereiche A1 ergeben, wenn der Stab 1 durch die Befestigungsmittel 6a, 6b, die auf die Auflagebereiche A3 wirken, gegen die Auflageteile 12a, 12b gedrückt werden. Der Querschnitt der Durchbrechung 3 ist in Fig. 4 allerdings rund dargestellt, sodass die Auflagebereiche A1 gegenüber den Befestigungsmitteln 6a, 6b zu liegen kommen. Ist der Querschnitt der Durchbrechung 3 hingegen erfindungsgemäss ausgebildet, zum Beispiel oval (Fig. 2c), so ergeben sich Auflagebereiche, die wie in Fig. 2d mit A1, A2 dargestellt, seitlich angeordnet sind.

Fig. 2a zeigt eine Seitenansicht eines Verbindungsgliedes 2, bestehend aus einem Aufnahmeteil 2a sowie einem Verbindungsteil 2b mit einer Durchbrechung 11 sowie einer Ausnehmung 5a. Die innere Begrenzungsfläche 4 der Durchbrechung ist im wesentlichen rund ausgestaltet, jedoch nicht kreisförmig. Aus Fig. 2c ist ersichtlich, dass die innere Begrenzungsfläche 4 mehrere gekrümmte Teilflächen 4a, 4b, 4c umfasst, die drei unterschiedliche Krümmungsradien r1, r2, r3 aufweisen, sodass sich im vorliegenden Ausführungsbeispiel ein eiförmiger (ovaler) verlauf der inneren Begrenzungsfläche 4 ergibt. Wird der Krümmungsradius r3 der Teilfläche 4c, die gegenüber der Austrittsöffnung der Ausnehmung 5a liegt, kleiner gewählt als der Krümmungsradius R1 des Stabes 1, so ergeben sich, wie in Fig. 2d dargestellt, zwei in Längsrichtung der Durchbrechung 3 verlaufende Auflagebereiche A1, A2, sodass sich zusammen mit dem Auflagebereich A3 des Befestigungsmittels 6a eine Dreipunktauflage des Stabes 1 ergibt.

Jede Gestaltungsform einer inneren Begrenzungsfläche 4 kann rillenförmige Vertiefungen 9 aufweisen, die zum Beispiel in Längsrichtung der Durchbrechung 3 und/oder in Querrichtung der Durchbrechung 3 verlaufen, oder in einer sonstigen Richtung. Eine vorteilhafte Ausgestaltung der Oberflächenstruktur der Begrenzungsfläche 4 lässt sich durch eine Vielzahl pyramidenförmiger Erhebungen erreichen. Ebenso kann es sich als vorteilhaft erweisen, den Stab 1 mit einer Oberflächenstruktur zu versehen, zum Beispiel mit einer bestimmten Rauhigkeit, wie dies zum Beispiel durch Sandstrahlen erzielt wird, oder zum Beispiel durch rillenförmige Vertiefungen.

Fig. 3 zeigt eine Vorrichtung zum Verbinden von mindestens zwei Wirbelknochen mit einem elastischen Stab 1. In den Wirbelknochen 7 werden Verankerungselemente 8 wie Knochenschrauben verankert und ein Verbindungsglied 2 mittels einer Klemmutter 10 an das Verankerungselement 8 befestigt. Der elastische Stab 1 ist mittels eines Befestigungsmittels 6a am Verbindungsglied 2 befestigt.

## Patentansprüche

1. Vorrichtung zum Verbinden von Wirbelknochen, welche Vorrichtung mindestens zwei Verbindungsglieder (2) umfasst, die jeweils ein Aufnahmeteil (2a) und ein Verbindungsteil (2b) aufweisen, wobei jedes Verbindungsglied (2) am Verbindungsteil (2b) mit Hilfe einer Knochenschraube (8) an dem jeweiligen zu verbindenden Wirbelknochen befestigbar ist, ferner mit einem elastischen Stab (1) kreisförmigen Querschnitts, welcher in eine Durchbrechung (3) im Aufnahmeteil (2b) jedes Verbindungsglieds (2) einbringbar und dort mittels eines Befestigungsmittels (6a,6b) befestigbar ist, welches durch eine quer zur Achse der Durchbrechung (3) angeordnete Ausnehmung (5a,5b) hindurch auf den Stab (1) wirkt,
dadurch gekennzeichnet, dass die Durchbrechung (3) eine im wesentlichen runde, innere Begrenzungsfläche (4) aufweist, die aus mehreren gekrümmten Teilflächen (4a, 4b, 4c) mit mindestens drei unterschiedlichen Krümmungsradien (r1, r2, r3) gebildet ist, wobei der Radius (r1) der Teilfläche (4a) auf der Seite der Ausnehmung (5a,5b) und der Radius (r2) der sich jeweils an diese Teilfläche (4a) anschliessenden Teilflächen (4b) grösser ist als der Radius (R1) des Stabes (1), wohingegen der Radius (r3) der Teilfläche (4c), die gegenüber der Ausnehmung (5a,5b) liegt, kleiner ist als der Radius (R1) des Stabes (1), sodass ein befestigter Stab (1) an zwei Auflagebereichen (A1,A2) an der inneren Begrenzungsfläche (4) sowie mit einem dritten Auflagebereich (A3) am Befestigungsmittel (6a,6b) aufliegt.

2. Vorrichtung nach Anspruch 1, dadurch gekennzeichnet, dass die innere Begrenzungsfläche (3) eiförmig ausgebildet ist.

3. Vorrichtung nach Anspruch 1 oder 2, dadurch gekennzeichnet, dass die innere Begrenzungsfläche (4) und/oder die Oberfläche des Stabes (1) rillenförmige Vertiefungen aufweist.

4. Vorrichtung nach Anspruch 3, dadurch gekennzeichent, dass die innere Begrenzungsfläche (4) sich kreuzende, rillenförmige Vertiefungen aufweist.

5. Vorrichtung nach Anspruch 4, dadurch gekennzeichnet, dass die rillenförmigen Vertiefungen sich gegenseitig etwa senkrecht kreuzend angeordnet sind, sodass die Begrenzungsfläche (4) eine Vielzahl pyramidenförmiger Erhebungen aufweist.

6. Vorrichtung nach einem der Ansprüche 1 bis 5, dadurch gekennzeichnet, dass das Aufnahmeteil (2a) mindestens eine weitere Ausnehmung (5b) aufweist für ein weiteres innerhalb der Durchbrechung (3) auf den Stab (1) wirkendes Befestigungsmittel (6b).

7. Vorrichtung nach Anspruch 6, dadurch gekennzeichnet, dass die Ausnehmungen (5a, 5b) bezogen auf die Länsgrichtung der Durchbrechung (3) axial beabstandet sind.

8. Vorrichtung nach einem der vorangehenden Ansprüche, dadurch gekennzeichnet, dass das Aufnahmeteil (2a) eine weitere Durchbrechung (12c) aufweist, die gegenüber den Ausnehmungen (5a, 5b) liegt, und dass diese Durchbrechung (12c) bezogen auf die Länsgrichtung der anderen Durchbrechung (3) axial durch zwei Auflageteile (12a, 12b) begrenzt ist.

## Claims

1. Apparatus for the connection of vertebrae, which apparatus comprises at least two connection members (2) which respectively have a receiving part (2a) and a connection part (2b), wherein connection member (2) at the connection part (2b) can be secured with the aid of a bone screw (8) to the respective vertebrae to be connected, furthermore having an elastic rod (1) of circular cross-section, which can be introduced in the receiving part (2b) of each connection member (2) and can be secured there by means of a fastener means (6a, 6b) which acts on the rod (1) through a cutout (5a, 5b) arranged transverse to the axis of the aperture (3), characterized in that the aperture (3) has a substantially round inner boundary surface (4), which is formed from a plurality of curved part surfaces (4a, 4b, 4c), with at least three different radii of curvature (r1, r2, r3), wherein the radius (r1) of the part surface (4a) at the side of the cutout (5a, 5b) and the radius (r2) of the part surfaces (4b) respectively adjoining this part surface (4a) is greater than the radius (R1) of the bar (1), whereas the radius (r3) of the part surface (4c) which lies opposite to the cutout (5a, 5b) is smaller than the radius (R1) of the rod (1), so that a secured rod (1) contacts at two contact regions (A1, A2) against the inner boundary surface (4) and also contacts on the fastener means (6a, 6b) with a third contact region (A3).

2. Apparatus in accordance with claim 1, characterised in that the inner boundary surface (3) is egg-shaped.

3. Apparatus in accordance with claim 1 or 2, characterised in that the inner boundary surface (3) and/or the surface of the bar (1) has groove-shaped recesses.

4. Apparatus in accordance with claim 3, characterized in that the inner boundary surface (3) has groove-shaped recessed which cross one another.

5. Apparatus in accordance with claim 4, characterised in that the groove-shaped recesses (9) are arranged so that they mutually cross approximately perpendicularly, so that the boundary surface (3) has a plurality of pyramidal elevations.

6. Apparatus in accordance with one of the claims 1 to 5, characterised in that the receiving part (2a) has at least one further cutout (5b) for a fastener means (6b) which acts on the rod (1) within the opening (3).

7. Apparatus in accordance with claim 6, characterised in that the cutouts (5a, 5b) are spaced apart in the axial direction with reference to the longitudinal direction of the aperture (3).

8. Apparatus in accordance with one of the preceding claims, characterised in that the receiving part (2a) has a further opening (12c) which lies opposite to the cutouts (5a, 5b), and in that this opening is bounded axially by two contact parts (12a, 12b) with reference to the longitudinal direction of the other aperture (3).

## Revendications

1. Dispositif pour connecter des vertèbres, ce dispositif comprenant au moins deux organes de connection (2) qui présentent chacun une partie de réception (2a) et une partie de connection (2b), où chaque organe de connection (2) peut être fixé à la partie de connection (2b) à l'aide d'une vis à os (8) à la vertèbre respectivement à connecter, avec en outre une tige élastique (1) d'une section transversale circulaire insérable dans un perçage (3) dans la partie de réception (2b) de chaque organe de connection (2) et pouvant y être fixé par un moyen de fixation (6a,6b) qui agit à travers un évidement (5a,5b) disposé transversalement à l'axe du perçage (3) sur la tige (1), caractérisé en ce que le perçage (3) présente une face de délimitation interne (4) sensiblement ronde qui est formée par plusieurs faces partielles courbées (4a,4b,4c) avec au moins trois rayons de courbure différents (r1,r2,r3), où le rayon (r1) de la face partielle (4a), sur le côté de l'évidement (5a,5b), et le rayon (r2) des faces partielles (4b) faisant suite respectivement à cette face partielle (4a) est plus grand que le rayon (R1) de la tige (1), tandis que le rayon (r3) de la face partielle (4c) qui se situe en face de l'évidement (5a,5b), est plus petit que le rayon (R1) de la tige (1) de telle sorte qu'une tige fixée (1) repose sur deux zones d'appui (A1,A2) à la face de délimitation interne (4) et, avec une troisième zone d'appui (A3) sur le moyen de fixation (6a,6b).

2. Dispositif selon la revendication 1, caractérisé en ce que la face de délimitation interne (3) est de forme ovoïde.

3. Dispositif selon la revendication 1 ou 2, caractérisé en ce que la face de délimitation interne (4) et/ou la surface de la tige (1) présente des creux en forme de rainure.

4. Dispositif selon la revendication 3, caractérisé en ce que la face de délimitation interne (4) présente des creux en forme de rainure qui se croisent.

5. Dispositif selon la revendication 4, caractérisé en ce que les creux en forme de rainure sont disposés de façon à se croiser mutuellement à peu près perpendiculairement de telle sorte que la face de délimitation (4) présente une multitude de surélévations en forme de pyramide.

6. Dispositif selon l'une des revendications 1 à 5, caractérisé en ce que la partie de réception (2a) présente au moins un évidement supplémentaire (5b) pour un moyen de fixation supplémentaire (6b) agissant à l'intérieur du perçage (3) sur la tige (1).

7. Dispositif selon la revendication 6, caractérisé en ce que les évidements (5a,5b), par rapport à la direction longitudinale du perçage (3), sont espacés axialement.

8. Dispositif selon l'une des revendications précédentes, caractérisé en ce que la partie de réception (2a) présente un perçage supplémentaire (12c) qui se situe en face des évidements (5a,5b), et que ce perçage (12c), par rapport à la direction longitudinale de l'autre perçage (3), est délimité axialement par deux parties d'appui (12a, 12b).
